# FASCICULE DE BREVET EUROPEEN

(11) **EP 2 306 911 B1**
(45) Date de publication et mention de la délivrance du brevet: **28.01.2015**
(21) Numéro de dépôt: 09781211.9
(22) Date de dépôt: 28.07.2009
(51) Int. Cl.: A61B 17/28, A61B 17/00, A61B 19/00

(54) **OUTIL CHIRURGICAL MODULAIRE**
MODULARES CHIRURGISCHES INSTRUMENT
MODULAR SURGICAL TOOL

(30) Priorité: 29.07.2008 FR 0855188
(43) Date de publication de la demande: 13.04.2011
(73) Titulaire: Universite Joseph Fourier - Grenoble 1, 38400 St. Martin d'Heres (FR)
(72) Inventeur: CINQUIN, Philippe, F-38330 St Nazaire Les Eymes (FR); AVILA VILCHIS, Juan, Carlos, Toluca 50130 (MX); ZEMITI, Nabil, F-34090 Montpellier (FR); VILCHIS GONZALEZ, Adriana, Herlinda, Toluca 50130 (MX)
(74) Mandataire: Regimbeau
(86) Numéro de dépôt international: PCT/EP2009/059771
(87) Numéro de publication internationale: WO 2010/012748

(56) Documents cités:
- US-A- 5 352 219
- US-A- 5 752 972
- US-A- 6 074 408
- US-A1- 2004 133 189
- TOSHIO TAKAYAMA ET AL: "Detachable-Fingered Hands for Manipulation of Large Internal Organs in Laparoscopic Surgery" ROBOTICS AND AUTOMATION, 2007 IEEE INTERNATIONAL CONFERENCE ON, IEEE, PI, 1 avril 2007 (2007-04-01), pages 244-249, XP031090816 ISBN: 978-1-4244-0601-2

## Description

### DOMAINE DE L'INVENTION

La présente invention concerne un outil chirurgical modulaire pour la chirurgie minimalement invasive.

### ARRIERE PLAN DE L'INVENTION

La chirurgie minimalement invasive est une approche chirurgicale visant à intervenir à l'intérieur du corps d'un patient en accédant aux organes sur lesquels une intervention doit être pratiquée en pratiquant un minimum d'ouvertures dans le corps.

A cet effet, le chirurgien effectue des incisions de petites dimensions dans le corps du patient, dans lesquelles il insère des trocarts destinés à permettre le passage d'instruments et d'appareils de visualisation, tels qu'un endoscope ou un laparoscope qui lui permettent d'observer la zone d'intervention.

Les trocarts permettant le passage d'appareils de visualisation s'appellent des « trocarts optiques » et ont, généralement, des diamètres intérieurs de 10 mm ou de 12 mm.

Les trocarts qui permettent le passage d'instruments de chirurgie sont dénommés « trocarts opérateurs » et ont, généralement, des diamètres intérieurs de 5 mm, étant entendu que des trocarts opérateurs de diamètre intérieur de 8 mm, de 10 mm ou de 12 mm peuvent aussi être utilisés si l'opération le nécessite.

En raison des avantages de cette technique chirurgicale pour le patient - notamment en termes de confort du patient, de rapidité de récupération postopératoire, de réduction de la durée d'hospitalisation -, de nombreux outils ont été développés pour permettre au chirurgien d'effectuer des gestes aussi précis qu'en chirurgie ouverte, dans laquelle il bénéficie d'un plus large accès à la région d'intervention.

Toutefois, les outils proposés à ce jour présentent encore des inconvénients qui entravent les progrès de la chirurgie minimalement invasive.

Parmi ces problèmes, le manque de dextérité ou la faible mobilité apportée par les outils actuels a été signalée par des médecins.

Des outils robotisés à mobilités internes - c'est-à-dire présentant différents degrés de liberté actionnables à l'intérieur du corps du patient - existent aujourd'hui en routine clinique (instruments du robot DaVinci) ou sous forme de prototype en laboratoire de recherche.

Pour permettre l'insertion de ces outils à travers les trocarts opérateurs conventionnels, l'actionnement des mobilités internes de ces outils se fait par moyen déporté (par exemple, des câbles, des biellettes, des fils à tension, des ressorts et des courroies, tirés par des actionneurs placés sur la partie proximale de l'instrument). Ces dispositifs d'actionnement engendrent un encombrement important à l'extérieur du patient, autour du chirurgien.

Une autre solution consiste à réaliser les mobilités internes en utilisant des actionnements directs (c'est-à-dire des mini-moteurs placés dans la partie distale de l'instrument).

Pour des raisons technologiques (compromis taille/puissance des moteurs), l'outil qui en découle est très encombrant et ne passe pas par les trocarts opérateurs classiques. L'utilisation de trocarts de plus grand diamètre devient nécessaire, ce qui supprime le caractère minimalement invasif et non traumatisant de la procédure chirurgicale.

Par ailleurs, la modularité dans la conception d'un outil ou d'un système médical a été prise en compte dans plusieurs travaux.

Par exemple, le brevet US 6,074,408 porte sur un instrument médical modulaire démontable afin de permettre un nettoyage plus facile et le remplacement d'un composant défaillant. Son actionnement repose sur une commande par câble interne.

Le document WO 98/49951 décrit quant à lui un instrument chirurgical modulaire autorisant une interchangeabilité de l'outil grâce à un système de cliquet à billes.

Le brevet US 7,150,751 porte sur la conception d'un raccord pour un outil modulaire.

Cependant, ces systèmes, bien que modulaires, ont été conçus pour un assemblage externe au corps du patient. En effet, l'outil est tout d'abord complètement assemblé et ensuite introduit dans le corps du patient pour effectuer la tâche pour laquelle il a été conçu. Ceci suppose soit l'utilisation de trocarts de diamètres importants pour le passage de ces outils - avec des conséquences néfastes sur les suites postopératoires, soit la conception d'outils peu encombrants pour pouvoir passer dans des trocarts de diamètre moindre, mais au détriment des degrés de liberté.

Plusieurs travaux ont proposé des architectures originales pour des outils médicaux tels que les endoscopes ou d'autres outils chirurgicaux, où les alliages à mémoire de forme sont parfois utilisés, en essayant toujours d'améliorer la dextérité du médecin lors de la réalisation du geste chirurgical.

Ainsi, un dispositif d'orientation à trois degrés de liberté et actionné par câbles fait l'objet du brevet US 6,685,698.

L'article de T. Takayama, T. Omata, T. Futami, H. Akamatsu, T. Ohya, K.Kojima, K.Takase and N. Tanaka ("Detachable-fingered hands for manipulation of large internal organs in laparoscopic surgery", Proc. of International Conference on Robotics and Automation (ICRA), pp. 244-249, 2007), décrit quant à lui l'assemblage, à l'intérieur de l'abdomen, de doigts mécaniques constituant une « main » dont la fonction est de serrer, de manipuler ou de pousser des grands organes internes. Toutefois, dans ce dispositif, le nombre de doigts est restreint à trois. Par ailleurs, leur actionnement se fait mécaniquement, par câbles, par une action manuelle du chirurgien exercée sur la partie proximale de l'instrument.

On comprend donc que le nombre de degrés de liberté que présentent en général les instruments existants pour la chirurgie minimalement invasive est trop faible, et que leur actionnement par câbles génère un encombrement important à l'extérieur du patient.

Un premier but de l'invention est donc de concevoir un outil modulaire à mobilités internes - c'est-à-dire qui puisse procurer au chirurgien tous les degrés de liberté dont il a besoin -, et qui puisse en même temps être assemblé et désassemblé facilement.

Un autre but de l'invention est de procurer un outil modulaire de dimensions suffisamment faibles pour qu'il puisse être inséré dans le corps humain à travers une ouverture de diamètre minimal, afin de limiter les conséquences postopératoires sur le patient.

Un autre but de l'invention est de permettre l'emploi d'une source de puissance non déportée, afin d'éviter un actionnement par câbles qui donne lieu à des contraintes mécaniques importantes.

Enfin, l'outil modulaire devrait pouvoir être adapté à l'utilisation dans un système robotisé.

### BREVE DESCRIPTION DE L'INVENTION

L'invention propose un système capable de réaliser les mobilités internes de l'outil avec un système modulaire qui utilise un actionnement direct tout en conservant le caractère non traumatisant et minimalement invasif de la procédure chirurgicale.

Conformément à l'invention, il est proposé un dispositif d'intervention chirurgicale minimalement invasive, comprenant une panoplie pour former un outil chirurgical modulaire, ladite panoplie comprenant une tige support et une pluralité de modules fonctionnels, dans laquelle chaque module fonctionnel possède des moyens de solidarisation à la tige support et/ou à un autre module fonctionnel et présente des dimensions appropriées pour l'insertion dans le corps d'un patient à travers un trocart, ledit dispositif étant caractérisé en ce que :
- chaque module fonctionnel présente au moins un degré de liberté en rotation ou en translation et/ou un embout fonctionnel tel qu'une pince, un crochet, un scalpel,
- la tige support et/ou les modules fonctionnels comprennent des moyens d'actionnement desdits modules fonctionnels selon leur degré de liberté et/ou leur fonction respective,
et en ce que ledit dispositif comprend une interface électromécanique apte à commander l'actionnement des modules fonctionnels assemblés formant l'outil.

De manière particulièrement avantageuse, chaque module fonctionnel présente au moins un degré de liberté en rotation ou en translation, et/ou un embout fonctionnel tel qu'une pince, un crochet, un scalpel.

Les moyens de solidarisation comprennent des parties mâles et femelles complémentaires et/ou des surfaces d'adhésion par contact magnétique.

De manière préférée, la tige support et/ou les modules fonctionnels comprennent des moyens pour alimenter en énergie et pour commander lesdits modules fonctionnels.

Selon un mode préféré de réalisation, la tige support présente un diamètre inférieur ou égal à 8 mm, et chaque module fonctionnel présente au moins une dimension inférieure ou égale à 12 mm.

Le dispositif comprend un premier trocart présentant un diamètre intérieur inférieur ou égal à 8 mm pour l'insertion de la tige et un deuxième trocart présentant un diamètre intérieur inférieur ou égal à 12 mm pour l'insertion des modules fonctionnels.

Selon un mode particulier de réalisation, le dispositif comprend des moyens d'imagerie de l'intérieur du corps du patient, tels qu'un endoscope ou un laparoscope.

Avantageusement, le dispositif comprend en outre des moyens pour manipuler chaque module fonctionnel dans le corps du patient.

Lesdits moyens de manipulation comprennent par exemple une pince solidaire des moyens d'imagerie, un aimant présentant une forme cylindrique creuse et apte à être monté sur les moyens d'imagerie sans en encombrer le champ visuel, ou une pince insérée à travers un troisième trocart.

Un autre objet de l'invention concerne un procédé d'assemblage d'un outil chirurgical modulaire, ledit outil comprenant au moins un module fonctionnel sur une tige support, ledit procédé étant caractérisé en ce qu'il comprend les étapes suivantes :
(a) introduction de la tige support dans un premier trocart installé au préalable dans le corps d'un patient ;
(b) passage, dans un deuxième trocart installé au préalable dans le corps d'un patient, d'un module fonctionnel,
(c) assemblage dudit module fonctionnel sur la tige support.

De manière avantageuse, l'étape (b) comprend le passage successif d'une pluralité de modules fonctionnels dont le dernier présente un embout fonctionnel, et l'étape (c) comprend l'assemblage successif desdits modules fonctionnels, de telle sorte que l'outil assemblé présente à son extrémité distale ledit embout fonctionnel.

Entre les étapes (b) et (c), le procédé comprend une étape de manipulation des modules fonctionnels pour les amener de la sortie du deuxième trocart vers la tige support.

### BREVE DESCRIPTION DES DESSINS

D'autres caractéristiques et avantages de l'invention ressortiront de la description détaillée qui va suivre, en référence aux dessins annexés sur lesquels :
- la figure 1 illustre de manière schématique l'installation des trocarts dans l'abdomen d'un patient dans le cadre d'une intervention chirurgicale minimalement invasive ;
- la figure 2 représente la panoplie de modules fonctionnels permettant d'assembler l'outil chirurgical modulaire conformément à l'invention ;
- la figure 3 représente deux modules fonctionnels particuliers pouvant être utilisés dans le cadre de l'invention ;
- la figure 4 illustre un exemple d'outil chirurgical assemblé ;
- la figure 5 illustre un autre exemple d'outil chirurgical assemblé ;
- la figure 6 illustre une autre variante de module fonctionnel ;
- la figure 7 illustre plusieurs configurations possibles du module fonctionnel illustré à la figure 6 ;
- la figure 8 illustre deux exemples d'embouts fonctionnels montés à l'extrémité du module fonctionnel représenté aux figures 6 et 7 ;
- la figure 9 représente le procédé d'assemblage de l'outil chirurgical modulaire ;
- la figure 10 illustre un dispositif auxiliaire permettant la manipulation des modules fonctionnels à l'intérieur du corps du patient ;
- la figure 11 illustre un autre dispositif permettant la manipulation des modules fonctionnels ;
- la figure 12 illustre deux variantes d'un module jonction permettant d'augmenter le nombre de degrés de liberté de l'outil chirurgical.

### DESCRIPTION DETAILLEE DE L'INVENTION

### Dispositif d'intervention chirurgicale

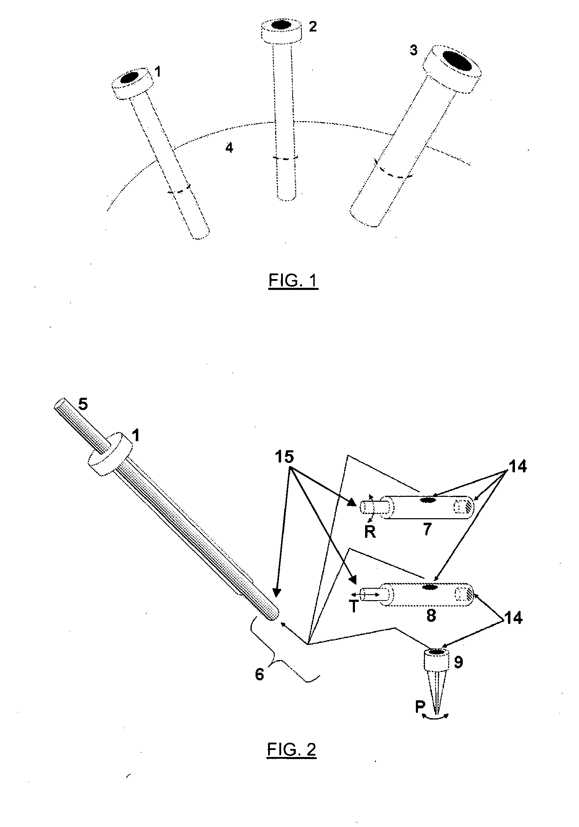

Lors d'une intervention chirurgicale minimalement invasive pratiquée dans la zone abdominale (ou laparoscopie), un trocart 1, un trocart 2, et un trocart 3 sont placés dans l'abdomen 4 d'un patient, comme illustré sur la figure 1.

Les trocarts 1 et 2 ont, généralement, un diamètre intérieur inférieur ou égal à 8 mm (trocart opérateur) et le trocart 3 (trocart optique) présente un diamètre intérieur inférieur ou égal à 12 mm.

Les trocarts 1 et 2 permettent l'utilisation d'outils chirurgicaux comme des pinces et le trocart 3 l'utilisation d'un laparoscope, par exemple.

La figure 2 montre le trocart 1 sectionné dans sa partie inférieure et la tige support 5 de l'outil chirurgical dont la partie distale 6 doit recevoir des modules fonctionnels à l'intérieur de l'abdomen du patient.

L'outil chirurgical modulaire conforme à l'invention est en effet assemblé à partir d'une panoplie de modules fonctionnels aptes à être solidarisés à la tige support ou à un autre module fonctionnel.

Par modulaire, on entend dans ce texte le fait que l'outil soit assemblé au cas par cas avec les degrés de liberté ou les fonctions souhaités par le chirurgien.

On précise par ailleurs que dans ce texte un degré de liberté est associé à une translation ou à une rotation ; les mouvements de fonctionnement de l'outil (par exemple ouverture et fermeture d'une pince) n'étant pas considérés comme des degrés de liberté.

La tige 5 sert de base pour l'assemblage/désassemblage d'une série de modules appartenant à l'une des classes suivantes : module présentant un degré de liberté en rotation (ou « module-rotation ») 7, module présentant un degré de liberté en translation (ou « module-translation ») 8 ou module présentant un embout fonctionnel, tel qu'un module-pince 9.

Bien sûr, l'embout fonctionnel peut être tout instrument dont le chirurgien peut avoir besoin lors de l'intervention, comme par exemple un crochet, un scalpel, une aiguille...

A cet effet, chaque module présente des moyens pour être solidarisé à la tige support ou à un autre module. Par exemple, chaque module peut présenter une forme femelle telle que l'orifice 14 représenté sur la figure 2, cette forme femelle étant apte à être emboîtée et maintenue sur une forme mâle 15 complémentaire de la tige support 5 ou d'un autre module fonctionnel (un arbre moteur, par exemple).

Les modules à embout fonctionnel, qui sont destinés à constituer l'extrémité de l'outil modulaire, peuvent présenter simplement un orifice 14.

Bien sûr, toute autre forme des moyens de solidarisation peut être envisagée (formes complémentaires mâle/femelle, clips, vis, moyens magnétiques, moyens électromécaniques, ...).

Par ailleurs, les moyens de solidarisation sont également conçus pour permettre la transmission de l'énergie et des commandes de fonctionnement à chacun des modules.

En particulier, l'outil est commandé à partir d'une interface électromécanique, du type interface haptique, au moyen de laquelle le chirurgien indique les mouvements souhaités et qui transmet, par le biais d'un contrôleur, les signaux de commande à chaque module fonctionnel.

La tige support 5 doit être conçue de telle sorte que sa partie interne contienne des supports de transmission (électrique, fluidique ou autre) pour l'alimentation en énergie et pour l'échange d'information et de signaux de commande entre les actionneurs, les capteurs et le contrôleur du système.

Par ailleurs, les parties proximale (externe à la cavité chirurgicale) et distale (dans la cavité chirurgicale) de la tige support 5 comprennent des contacteurs ou des connecteurs assurant les liaisons entre la tige et les modules fonctionnels (actionneurs) d'une part, et la tige support et le contrôleur d'autre part.

Selon une variante, on peut imaginer que chacun des modules fonctionnels dispose de ses propres moyens d'alimentation en énergie, par exemple sous la forme de batteries miniatures.

Aussi, selon un autre mode de réalisation de l'invention, la tige support 5 est une simple tige passive et l'alimentation en énergie, l'échange d'information et de signaux de commande entre les actionneurs, les capteurs et le contrôleur du système se fait par voie sans fil (radio fréquence, ultrason, Bluetooth, Wifi, induction, magnétique, etc.).

L'orifice 14 servant au montage des modules peut être situé sur la face la plus appropriée de chaque module.

Si, comme sur la figure 2, le module fonctionnel a une forme cylindrique, par exemple, l'orifice 14 peut être placé dans la base ou dans la surface latérale du cylindre. Si le module fonctionnel est un cube, l'orifice 14 peut être placé sur l'une des cinq surfaces du cube ne contenant pas l'axe moteur du module.

De manière alternative, on n'utilisera pas des parties mâles et femelles complémentaires mais des surfaces d'adhésion par contact magnétique.

La longueur de chaque module peut être supérieure à 12 mm ; cependant, sa largeur sera inférieure à 12 mm car l'assemblage de l'outil est basé sur l'utilisation du trocart 3 de diamètre intérieur inférieur ou égal à 12 mm afin de faire passer les modules définissant l'architecture souhaitée, vers l'intérieur de l'abdomen 4.

Sur chaque module-rotation 7 ou sur chaque module-translation 8, il est possible de monter/démonter d'autres modules sans aucune restriction de classe.

Le module-pince 9 ne reçoit pas d'autres modules, il peut être monté sur la tige support 5 en tant que premier et dernier module, mais aussi sur un module-rotation 7 ou sur un module-translation 8.

L'architecture de l'outil assemblé peut donc avoir le nombre de degrés de liberté que l'on souhaite, afin de procurer la meilleure dextérité au médecin pour réaliser son geste chirurgical.

Selon une variante, on peut envisager un module fonctionnel à plusieurs degrés de liberté et intégrant en outre un embout fonctionnel.

Ce système de classe « module-unique » 10 aura aussi une largeur inférieure à 12 mm et son architecture dépendra des besoins médicaux.

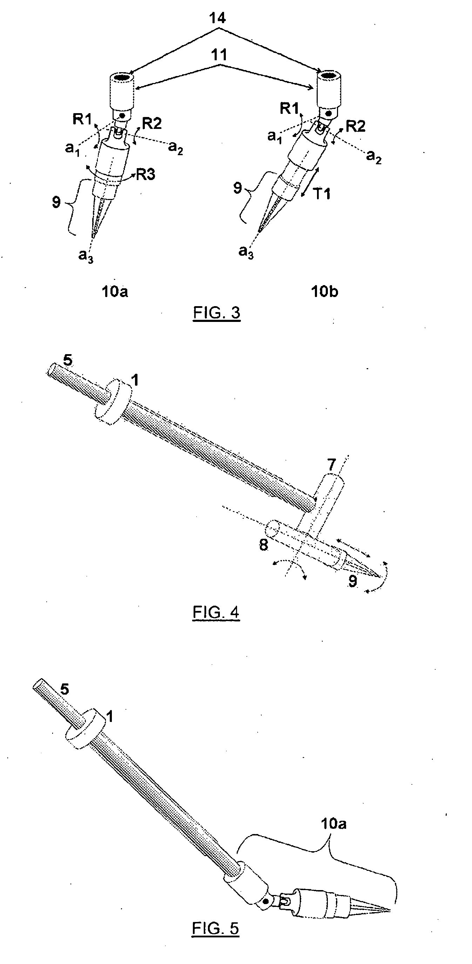

La figure 3 montre le principe général de deux modules de la classe module-unique 10 ; cependant, la conception de cette classe de modules n'est pas restreinte à ces deux exemples de module-unique.

Le premier module 10a possède une configuration du type Rotation-Rotation-Rotation-Pince où R1, R2 et R3 sont des rotations qui peuvent être réalisées par rapport aux axes a1, a2 et a3, respectivement.

Le deuxième module 10b a une configuration du type Rotation-Rotation-Translation-Pince où R1 et R2 sont les rotations réalisées par rapport aux axes a1, a2, respectivement, et T1 est une translation effectuée le long de l'axe a3.

Les parties 11 dans la figure 3 sont celles qui seront montées sur la tige support 5 grâce aux orifices 14. Il est possible d'observer sur la figure 3 un module-pince 9 comme partie finale de chaque module-unique 10.

En fonction des besoins chirurgicaux et/ou du degré de dextérité requis lors d'une opération, le système peut être mis à jour ou reconfiguré à la demande du chirurgien, c'est-à-dire que des modules-rotation 7 ou des modules-translation 8 peuvent être rajoutés ou enlevés

La figure 4 illustre un exemple de montage à deux degrés de liberté, constitué d'un module-rotation 7, d'un module-translation 8 et d'un module-pince 9.

La figure 5 illustre un module 10a de classe « module-unique » monté sur la tige support 5.

La récupération des modules de classe « module-unique » 10 se fera après l'avoir démonté de la tige support 5, par un des moyens mentionnés plus loin dans ce document.

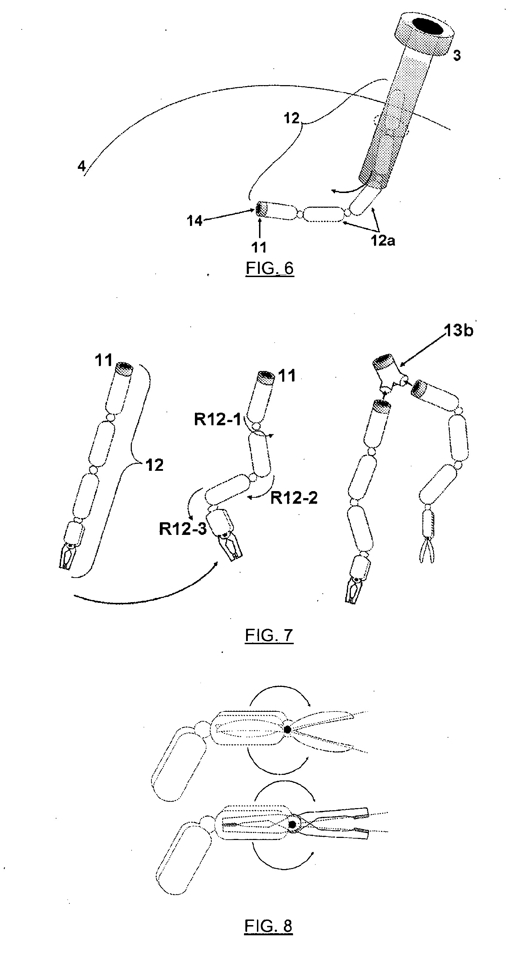

Selon une autre variante de la panoplie, on peut prévoir une classe particulière de module unique 12 constitué d'une série de sous-modules indépendants 12a comme illustré à la figure 6.

Ce module 12 est aussi envoyé vers la cavité chirurgicale 4 par le trocart 3. La partie initiale 11 de cette série de sous-modules est la partie qui sera montée sur la tige support (non montrée sur cette figure).

Une fois assemblé sur la tige support, ce module 12 pourra prendre une configuration particulière parmi un certain nombre de configurations possibles a priori, en fonction des besoins chirurgicaux.

Ainsi, la figure 7 illustre le module 12 dans une configuration c1 avant et après trois rotations R12-1, R12-2 et R12-3 des sous-modules.

Une deuxième configuration c2 du module 12 est aussi montrée à la figure 7 où le module 12 a pris une configuration à deux branches, la première portant une pince à la fin de la série de sous-modules et la deuxième portant des ciseaux.

Le chirurgien intervient uniquement dans le montage du module 12 sur la tige support 5.

Un module jonction a été conçu afin d'augmenter le nombre de montages des modules à l'intérieur de la cavité chirurgicale.

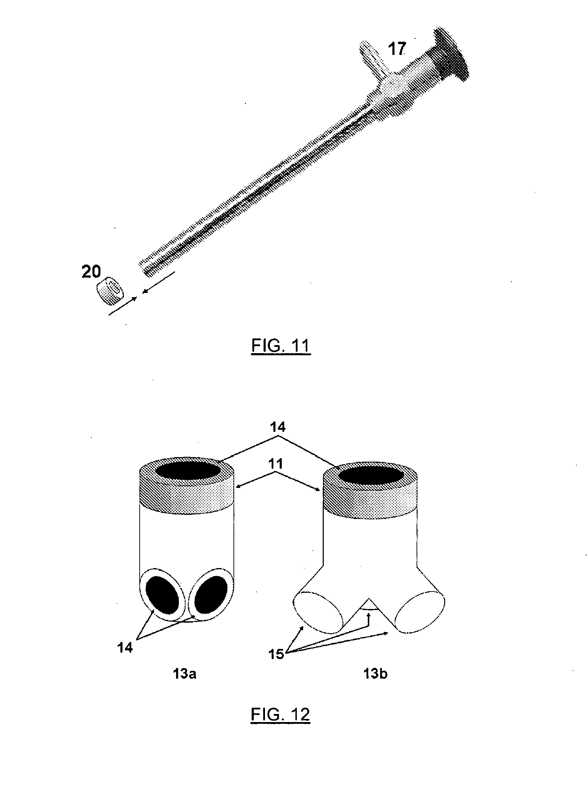

La figure 12 illustre deux versions du module jonction: un module femelle-femelle 13a et un module femelle-mâle 13b.

Le module jonction 13a ou 13b peut recevoir les différentes classes de modules 7,8,9, 10 ou 12.

En effet, l'utilisation du module jonction permet un montage multiple comme celui de deux modules 12 illustré dans la partie droite de la figure 7 où un module jonction 13b (monté au préalable dans la tige support 5) reçoit un premier module 12 portant une pince et un deuxième module 12 portant des ciseaux.

L'utilisation du module jonction permet donc d'augmenter le nombre d'instruments à l'intérieur de la cavité chirurgicale sans pourtant augmenter le nombre d'orifices chirurgicaux.

La partie gauche de la figure 7 illustre le module 12 avant et après trois rotations R12-1, R12-2 et R12-3 des sous-modules, ce qui définit un changement de configuration. Ces rotations sont effectuées à l'intérieur de la cavité chirurgicale après montage du module 12 dans la tige support 5 ou dans le module jonction ; elles s'effectuent grâce à une commande réalisée à l'extérieur de la cavité chirurgicale.

Dans ce cas, le chirurgien intervient uniquement dans le montage du module 12 sur la tige support 5 ou du module jonction sur la tige support 5 et des modules 12 sur le module jonction.

Par rapport au module unique 10, le module 12 possède un plus grand nombre de degrés de liberté grâce au nombre de sous-modules indépendants 12a qui le constituent et qui peuvent être choisis à l'avance par le médecin ; ce module 12 est donc redondant. Ceci représente une augmentation du degré de dextérité de l'outil chirurgical et permet d'avoir les mobilités souhaitées selon l'opération, sans avoir à changer d'instrument ou à rajouter des modules

Les pinces, ciseaux, etc. du module 12 sont des outils dépliables formant aussi un sous-module, comme il est illustré dans la figure 8.

### Procédé d'assemblage de l'outil modulaire

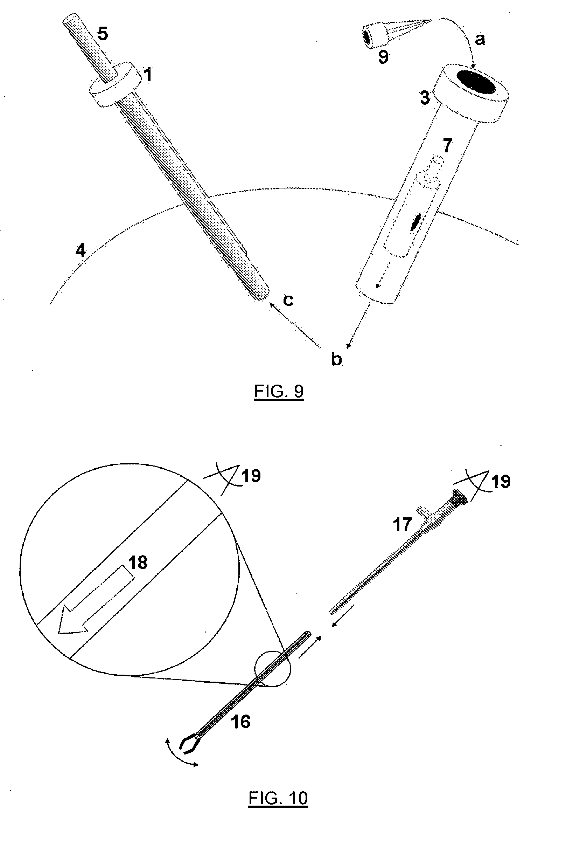

La figure 9 illustre les différentes étapes nécessaires pour assembler l'outil à l'intérieur de l'abdomen.

Dans l'étape (a) les modules sont passés par le trocart 3 où il est possible d'observer, en pointillées, un module-rotation 7 passant par le trocart 3 et un module-pince 9 prêt à être inséré dans le trocart 3.

Le passage des modules vers l'intérieur de l'abdomen du patient pour le montage de l'outil et leur récupération après démontage, peuvent se faire en utilisant des moyens mécaniques, magnétiques, pneumatiques ou autres.

Les modules peuvent ainsi être envoyés vers l'intérieur de l'abdomen par gravité où ils seront récupérés pour leur assemblage.

L'étape (b) correspond à la récupération des modules à l'intérieur de l'abdomen en utilisant, par exemple, un autre outil chirurgical placé dans le trocart 2, qui n'est pas représenté sur cette figure.

Selon un autre mode de réalisation de l'invention, illustré à la figure 10, on utilise un dispositif auxiliaire 16 comprenant des moyens de préhension pour manipuler des différents modules.

Le dispositif auxiliaire 16 est par exemple adapté au laparoscope 17 sans obstruer le champ visuel 18 de la caméra.

En effet, ce dispositif auxiliaire 16 doit avoir une architecture bien étudiée afin de faciliter le repérage et la manipulation des modules, par la région sans interférence du champ visuel 18.

Pour la phase d'assemblage de l'outil chirurgical, le dispositif auxiliaire 16 est monté sur le laparoscope 17 avant de faire passer le premier module vers l'intérieur de l'abdomen et démonté après avoir fini l'assemblage de l'outil pour ne pas gêner la vue disponible pour le médecin.

Dans l'étape (c), les modules sont assemblés l'un après l'autre sur la tige support 5, en terminant par un module-pince 9.

Une fois assemblé, l'outil est prêt pour la mise en oeuvre d'une procédure chirurgicale définie par le chirurgien et qui ne fait pas l'objet de la présente invention.

Le nombre et la classe de modules à utiliser ainsi que l'ordre de montage ou la configuration du module dépendent de la dextérité souhaitée par le médecin et de l'espace interne disponible dans l'abdomen du patient.

Pour actionner les différents modules, l'utilisation de n'importe quelle sorte d'énergie peut être envisagée.

La sélection du type d'énergie à utiliser dépendra des contraintes médicales pour chaque application et de la disponibilité technologique du moment.

La tige support 5 est conçue en fonction de la nature de l'énergie sélectionnée afin d'assurer l'alimentation en énergie pour tous les modules définissant l'architecture finale de l'outil chirurgical.

Comme expliqué plus haut, le moyen utilisé pour fournir de l'énergie et d'échanger les informations et les signaux de commande entre le contrôleur et les modules fonctionnels peut être un moyen conventionnel (fils électriques, pneumatique, etc.) ou bien un moyen sans fil (radio fréquence, induction, magnétique, Bluetooth, ultrason, etc.).

Dans le cas d'une transmission conventionnelle, le moyen de solidarisation de chaque module fonctionnel est donc conçu de telle sorte qu'il reçoive et transmette (avec le contrôleur et avec tous les modules) les informations et les signaux de commande ainsi que l'énergie nécessaire.

Contrairement aux outils de l'art antérieur, l'outil conforme à l'invention peut donc s'affranchir d'un actionnement par câbles, ce qui minimise l'encombrement généré autour du chirurgien.

Le désassemblage de l'outil est effectué en démontant un par un les modules utilisés. Les modules sont ensuite retirés de l'intérieur de l'abdomen à travers le trocart 3.

Par exemple, le dispositif auxiliaire 16 est monté sur le laparoscope 17 avant de commencer le désassemblage de l'outil chirurgical et démonté après avoir récupéré tous les modules.

Un autre moyen pour récupérer les modules de l'intérieur de l'abdomen du patient est un aimant creux 20 monté sur le laparoscope, comme illustré à la figure 11.

L'orifice de l'aimant permet de libérer le champ visuel du laparoscope 17.

Ce système permet de récupérer les modules un par un par simple contact avec la face adéquate pour garantir son passage par le trocart 3.

Il est possible aussi d'utiliser d'autres moyens pour déposer et pour récupérer les modules de l'intérieur de l'abdomen ainsi que pour les monter/démonter, comme par exemple les systèmes à verrou, les systèmes de fixation à billes, les systèmes de clipsage ou n'importe quel autre type de système de fixation ayant des qualités telles que la rapidité de montage/démontage, la facilité d'utilisation et la sécurité.

Parmi les moyens de récupération des modules, un sac endoscopique, utilisé habituellement pour extraire de la cavité chirurgicale une partie d'un organe séparée par ablation, peut aussi être utilisé.

Les matériaux utilisés pour la fabrication des différents éléments tels que la tige support 5, les modules 7, 8, 9, 10 12 et 13a ou 13b, le dispositif auxiliaire 16, l'aimant 20 ou un autre dispositif de fixation servant à la mise en oeuvre de l'invention, sont compatibles avec les conditions de stérilisabilité et de sécurité requises dans une salle d'opérations.

De manière particulièrement avantageuse, l'outil modulaire qui vient d'être décrit peut être utilisé en relation avec le système de positionnement robotisé faisant l'objet de la demande internationale WO03/094579. L'extrémité proximale de la tige support 5 est alors solidarisée à un moyen de support approprié. Ainsi, le chirurgien peut bénéficier des fonctionnalités et du faible encombrement d'un tel système robotisé, tout en profitant de tous les degrés de liberté autorisés par l'outil modulaire.

Bien sûr, les dimensions des trocarts ont été données à titre d'exemple, étant entendu que des trocarts de plus grand diamètre peuvent être utilisés pour permettre le passage de modules plus volumineux.

Enfin, l'invention a été décrite dans le cadre de la laparoscopie, où la cavité chirurgicale est l'abdomen, mais il va de soi qu'elle s'applique à toutes les interventions envisageables en chirurgie minimalement invasive.

## Revendications

1. Dispositif d'intervention chirurgicale minimalement invasive, comprenant une panoplie pour former un outil chirurgical modulaire, ladite panoplie comprenant une tige support (5) et une pluralité de modules fonctionnels (7, 8, 9, 10, 12), dans laquelle chaque module fonctionnel possède des moyens (14, 15) de solidarisation à la tige support (5) et/ou à un autre module fonctionnel et présente des dimensions appropriées pour l'insertion dans le corps d'un patient à travers un trocart, dans lequel :
- chaque module fonctionnel (7, 8, 9, 10, 12) présente au moins un degré de liberté en rotation ou en translation et/ou un embout fonctionnel tel qu'une pince, un crochet, un scalpel,
- la tige support (5) et/ou les modules fonctionnels (7, 8, 9, 10, 12) comprennent des moyens d'actionnement desdits modules fonctionnels selon leur degré de liberté et/ou leur fonction respective,
- ledit dispositif comprend une interface électromécanique apte à commander l'actionnement des modules fonctionnels assemblés formant l'outil,
ledit dispositif étant **caractérisé en ce que** la panoplie comprend au moins un module jonction (13a, 13b) comprenant des moyens de solidarisation (14, 15) à la tige support (5) et à au moins deux modules fonctionnels (7, 8, 9, 10, 12).

2. Dispositif selon la revendication 1, **caractérisé en ce que** les moyens de solidarisation comprennent des parties mâles et femelles complémentaires et/ou des surfaces d'adhésion par contact magnétique.

3. Dispositif selon l'une des revendications 1 ou 2, **caractérisé en ce que** la tige support (5) et/ou les modules fonctionnels (7, 8, 9, 10, 12) comprennent des moyens pour alimenter en énergie lesdits modules fonctionnels.

4. Dispositif selon l'une des revendications 1 à 3, **caractérisé en ce que** la tige support (5) présente un diamètre inférieur ou égal à 8 mm, et **en ce que** chaque module fonctionnel (7, 8, 9, 10, 12) présente au moins une dimension inférieure ou égale à 12 mm.

5. Dispositif selon l'une des revendications 1 à 4, **caractérisé en ce qu'**il comprend des moyens d'imagerie de l'intérieur du corps du patient, tels qu'un endoscope ou un laparoscope.

6. Dispositif selon l'une des revendications 1 à 5, **caractérisé en ce qu'**il comprend en outre des moyens (16, 20) pour manipuler chaque module fonctionnel dans le corps du patient.

7. Dispositif selon la revendication 6 dans sa relation de dépendance vis-à-vis de la revendication 5, **caractérisé en ce que** lesdits moyens de manipulation comprennent une pince solidaire des moyens d'imagerie.

8. Dispositif selon la revendication 7, **caractérisé en ce que** les moyens de manipulation comprennent un aimant présentant une forme cylindrique creuse et apte à être monté sur les moyens d'imagerie sans en encombrer le champ visuel.

9. Dispositif selon la revendication 8, **caractérisé en ce que** lesdits moyens de manipulation comprennent une pince insérée à travers un troisième trocart (2).

10. Dispositif selon l'une des revendications 1 à 9, **caractérisé en ce qu'**il comprend un premier trocart (1) présentant un diamètre intérieur inférieur ou égal à 8 mm pour l'insertion de la tige et un deuxième trocart (3) présentant un diamètre intérieur inférieur ou égal à 12 mm pour l'insertion des modules fonctionnels.

## Patentansprüche

1. Vorrichtung für minimal invasiven chirurgischen Eingriff, die einen Komponentensatz zum Zusammenbauen eines modularen chirurgischen Instruments aufweist, wobei der Komponentensatz einen Trägerschaft (5) und mehrere Funktionsmodule (7,8,9,10,12) aufweist, wobei jedes Funktionsmodul Verbindungseinrichtungen (14,15) zum Verbinden mit dem Trägerschaft (5) und/oder mit einem anderen Funktionsmodul aufweist und geeignete Abmessungen hat, um über einen Trokar in den Körper eines Patienten eingeführt zu werden, wobei
- jedes Funktionsmodul (7,8,9,10,12) mindestens einen Rotations- oder Translations-freiheitsgrad und/oder ein funktionales Ende, beispielsweise eine Zange, einen Haken, ein Skalpell, aufweist
- der Trägerschaft (5) und/oder die Funktionsmodule (7,8,9,10,12) Betätigungseinrichtungen zum Betätigen der Funktionsmodule gemäß ihrem Freiheitsgrad und/oder ihrer jeweiligen Funktion aufweisen,
- die Vorrichtung eine elektromechanische Schnittstelle aufweist, die die Betätigung der Funktionsmodule, aus denen das Instrument zusammengebaut ist, steuern kann,
wobei die Vorrichtung **dadurch gekennzeichnet ist, dass** der Komponentensatz mindestens ein Koppelmodul (13a,13b) aufweist, das Verbindungseinrichtungen (14,15) zum Verbinden mit dem Trägerschaft (5) und mit mindestens zwei Funktionsmodulen (7,8,9,10,12) aufweist.

2. Vorrichtung nach Anspruch 1, **dadurch gekennzeichnet, dass** die Verbindungseinrichtungen komplementäre männliche und weibliche Teile und/oder magnetisch haftende Kontaktflächen aufweisen.

3. Vorrichtung nach einem der Ansprüche 1 oder 2, **dadurch gekennzeichnet, dass** der Trägerschaft (5) und/oder die Funktionsmodule (7,8,9,10,12) Einrichtungen zum Versorgen der Funktionsmodule mit Energie aufweisen.

4. Vorrichtung nach einem der Ansprüche 1 bis 3, **dadurch gekennzeichnet, dass** der Trägerschaft (5) einen Durchmesser von kleiner oder gleich 8mm aufweist, und dadurch, dass jedes Funktionsmodul (7,8,9,10,12) mindestens eine Abmessung von kleiner oder gleich 12mm aufweist.

5. Vorrichtung nach einem der Ansprüche 1 bis 4, **dadurch gekennzeichnet, dass** sie Abbildungseinrichtungen zum Abbilden des Inneren des Körpers des Patienten aufweist, beispielsweise ein Endoskop oder ein Laparoskop.

6. Vorrichtung nach einem der Ansprüche 1 bis 5, **dadurch gekennzeichnet, dass** sie ferner aufweist: Manipulationseinrichtungen (16,20) zum Manipulieren jedes Funktionsmoduls im Körper des Patienten.

7. Vorrichtung nach Anspruch 6 in Abhängigkeitsbeziehung mit Anspruch 5, **dadurch gekennzeichnet, dass** die Manipulationseinrichtungen eine an den Abbildungseinrichtungen angebrachte Zange aufweisen.

8. Vorrichtung nach Anspruch 7, **dadurch gekennzeichnet, dass** die Manipulationseinrichtungen einen Elektromagneten in Form eines Hohlzylinders aufweisen, der an den Abbildungseinrichtungen angebracht werden kann, ohne dort das Sehfeld zu beeinträchtigen.

9. Vorrichtung nach Anspruch 8, **dadurch gekennzeichnet, dass** die Manipulationseinrichtungen eine über einen dritten Trokar (2) eingeführte Zange aufweisen.

10. Vorrichtung nach einem der Ansprüche 1 bis 9, **dadurch gekennzeichnet, dass** sie aufweist: einen ersten Trokar (1) eines Innendurchmessers von kleiner oder gleich 8mm zum Einführen des Schafts; und einen zweiten Trokar (3) eines Innendurchmessers von kleiner oder gleich 12mm zum Einführen der Funktionsmodule.

## Claims

1. A minimally invasive surgical intervention device, comprising a set for forming a modular surgical tool, said set comprising a support rod (5) and a plurality of functional modules (7, 8, 9, 10, 12), wherein each functional module has connecting means (14, 15) to the support rod (5) and/or to another functional module and has appropriate dimensions for insertion into the body of a patient via a trocar, wherein:
- each functional module (7, 8, 9, 10, 12) has at least one degree of freedom in rotation or translation and/or a functional end-piece such as forceps, a hook, a scalpel,
- the support rod (5) and/or the functional modules (7, 8, 9, 10, 12) comprise actuation means of said functional modules according to their degree of freedom and/or their respective function,
- said device comprises an electromechanical interface capable of controlling the actuation of the installed functional modules forming the tool,
said device being **characterized in that** the set comprises at least one junction module (13a, 13b) comprising connecting means (14, 15) to the support rod (5) and at least two functional modules (7, 8, 9, 10, 12).

2. The device according to Claim 1, **characterized in that** the attachment means comprise complementary male and female parts and/or adhesion surfaces by magnetic contact.

3. The device according to one of Claims 1 or 2, **characterized in that** the support rod (5) and/or the functional modules (7, 8, 9, 10, 12) comprise means for powering said functional modules.

4. The device according to one of Claims 1 to 3, **characterized in that** the support rod (5) has a diameter less than or equal to 8 mm, and **in that** each functional module (7, 8, 9, 10, 12) has at least one dimension less than or equal to 12 mm.

5. The device according to one of Claims 1 to 4, **characterized in that** it comprises imaging means of the interior of the body of the patient, such as an endoscope or a laparoscope.

6. The device according to one of Claims 1 to 5, **characterized in that** it further comprises means (16, 20) for handling each functional module in the body of the patient.

7. The device according to Claim 6 in its dependence relation vis-à-vis Claim 5, **characterized in that** said handling means comprise forceps connected to the imaging means.

8. The device according to Claim 7, **characterized in that** the handling means comprise a magnet having a hollow cylindrical shape and capable of being mounted on the imaging means without blocking the visual field.

9. The device according to Claim 8, **characterized in that** said handling means comprise forceps inserted via a third trocar (2).

10. The device according to one of Claims 1 to 9, **characterized in that** it comprises a first trocar (1) having an inner diameter less than or equal to 8 mm for the insertion of the rod and a second trocar (3) having an inner diameter less than or equal to 12 mm for inserting the functional modules.
